(19) Europäisches Patentamt

European Patent Office

Office européen des brevets

(11) Veröffentlichungsnummer: **0 475 272 B1**

(12) **EUROPÄISCHE PATENTSCHRIFT**

(45) Veröffentlichungstag der Patentschrift: **11.01.95**

(21) Anmeldenummer: **91114973.0**

(22) Anmeldetag: **05.09.91**

(51) Int. Cl.⁶: **C07C 50/02**, C07C 46/08

(54) Verfahren zur Herstellung von 2,3,5-Trimethyl-p-benzochinon.

(30) Priorität: **14.09.90 DE 4029198**

(43) Veröffentlichungstag der Anmeldung:
**18.03.92 Patentblatt 92/12**

(45) Bekanntmachung des Hinweises auf die
Patenterteilung:
**11.01.95 Patentblatt 95/02**

(84) Benannte Vertragsstaaten:
**CH DE FR GB LI**

(56) Entgegenhaltungen:
**EP-A- 0 167 153**
**EP-A- 0 294 584**
**EP-A- 0 387 820**

(73) Patentinhaber: **BASF Aktiengesellschaft
Carl-Bosch-Strasse 38
D-67063 Ludwigshafen (DE)**

(72) Erfinder: **Bockstiegel, Bernhard, Dr.
Chenoverstrasse 11
W-6703 Limburgerhof (DE)**
Erfinder: **Hoercher, Ulrich, Dr.
Kolpingstrasse 15
W-6800 Mannheim 1 (DE)**
Erfinder: **Laas, Harald, Dr.
Sohlstrasse 105
W-6701 Maxdorf (DE)**
Erfinder: **Jessel, Barbara, Dr.
Hochfeldstrasse 26
W-6700 Ludwigshafen (DE)**
Erfinder: **Grafen, Paul, Dr.
Suedtiroler Ring 20
W-6719 Weisenheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein verbessertes Verfahren zur Herstellung von 2,3,5-Trimethyl-p-benzochinon (im folgenden TMC genannt) durch Oxidation von einem Trimethylphenol, insbesondere 2,3,6-Trimethylphenol (im folgenden TMP genannt), mit gasförmigem Sauerstoff in Anwesenheit eines Kupferhalogenid enthaltenden Katalysators.

TMC ist ein wichtiges Zwischenprodukt für die Herstellung von Trimethylhydrochinon, einem essentiellen Vorprodukt für die Synthese von $\alpha$-Tocopherol (Vitamin E).

In Anbetracht seiner großen Bedeutung hat es nicht an Versuchen gefehlt, ein besonders vorteilhaftes Verfahren zur Herstellung von TMC zu finden.

So sind bereits zahlreiche Methoden für die Oxidation von TMP und anderen Phenolen zu den entsprechenden Benzochinonen mit Sauerstoff als Oxidationsmittel bekannt.

Gemäß dem Verfahren des deutschen Patents 2 221 624 werden hierfür Kupfer(II)-chlorid als Katalysator und Dimethylformamid (DMF), Ethylenglykolmonomethylether oder Aceton, gegebenenfalls in Kombination mit Wasser, als Lösungsmittel verwendet.

Die Oxidation von TMP zu TMC läßt sich auch anschließend an die Herstellung von TMP aus 2,3,6-Trimethyl-2-cyclohexen-1-on als Eintopfreaktion durchführen. Gemäß dem Verfahren der EP 93 880 werden hierfür niedere Alkohole (insbesondere Isopropanol) als Lösungsmittel verwendet; gemäß der EP 35 635 werden DMF, Acetonitril, Diethylenglykolmonomethylether oder Diethylenglykoldimethylether als Lösungsmittel bevorzugt. Oxidiert wird bei beiden Verfahren mit Luft oder Sauerstoff unter Katalyse von Kupfer(II)-halogeniden.

Gemaß dem Verfahren des deutschen Patents 2 827 552 wird für die Oxidation von Phenol zu p-Benzochinon Kupfer(I)- oder Kupfer(II)-chlorid, dem elementares Metall sowie ein Alkalimetall- oder Erdalkalimetallhalogenid zugesetzt wurde, als Katalysator verwendet. Als Lösungsmittel werden Methanol, Acetonitril oder DMF genannt. Gemäß den Verfahren von EP 70 665 und EP 107 427 werden Kupfer(II)-halogenid mit einer Alkalimetall- bzw. Erdalkalimetallbase als Promotor als Katalysator und Acetonitril als Lösungsmittel verwendet.

In der DE 2 444 234 wird für die Oxidation von TMP die Anwendung eines Sauerstoffpartialdrucks bis zu 52 bar beschrieben, wobei dem Kupferkatalysator in DMF ein Thiocyanat, Cyanat oder Cyanid zugesetzt wird.

In EP 127 888 wird ein Verfahren zur Herstellung von TMC durch Oxidation von TMP mit $O_2$ in einem aus Wasser und einem aliphatischen $C_4$- bis $C_{10}$-Alkohol bestehenden zweiphasigen Reaktionsmedium und in Gegenwart eines Kupfer-Alkalimetall-Halogen-Komplexes der allgemeinen Formel $M_l[Cu(II)_mX_n]_p$, in der M ein Alkalimetall oder Ammonium, X ein Halogenatom, l eine ganze Zahl von 1 bis 3, m und p die Zahlen 1 oder 2, und n eine ganze Zahl von 3 bis 6, wobei gilt $1 + 2$ mp = n.p, beschrieben. Gemäß diesem Verfahren werden gute Ausbeuten an TMC erhalten.

Aus EP 167 153 ist weiterhin ein Verfahren zur Herstellung von TMC bekannt, welches mit dem vorher beschriebenen Verfahren im wesentlichen identisch ist, nur daß dem Katalysator zusätzlich Kupfer(I)-hydroxid oder Kupfer(I)-chlorid zugefügt wird und ein langsames Zutropfen der alkoholischen TMP-Lösung zu der wäßrigen Katalysatorlösung als wesentlich angegeben wird.

Aus EP 294 584 ist ein Verfahren für die Herstellung von TMC durch Oxidation von TMP mit $O_2$ in Gegenwart eines Katalysators, bestehend aus Kupfer(II)-chlorid und Lithiumchlorid, und in einem zweiphasigen Reaktionsmedium, bestehend aus Wasser und einer Mischung von einem aromatischen Kohlenwasserstoff und einem niederen aliphatischen Alkohol mit 1 bis 4 C-Atomen bekannt.

Eine weitere Variante für die Herstellung von TMC durch Oxidation von TMP mit $O_2$ in einem einphasigen oder zweiphasigen Reaktionsmedium wird in EP 369 823 beschrieben. Als Katalysator wird die Kombination aus einer Kupferverbindung, bevorzugt Kupfer(II)-chlorid, und einer stickstoffhaltigen Verbindung, vorzugsweise einem Hydroxylamin, einem Oxim, einem Amin oder deren Salzen mit anorganischen Säuren, verwendet. Als Lösungsmittel dienen aliphatische Alkohole mit 1 bis 10 C-Atomen oder Mischungen aus aliphatischen Alkoholen mit 1 bis 6 C-Atomen und aromatischen Kohlenwasserstoffen.

Ein Vorteil der Verfahren gemäß EP 127 888, EP 167 153, EP 294 584 und EP 369 823 ist die leichte Abtrennbarkeit des Katalysators vom Reaktionsprodukt durch das Arbeiten im zweiphasigen Lösungsmittelsystem Alkohol/Wasser bzw. Alkohol und aromatischer Kohlenwasserstoff/Wasser.

Nachteilig an dem Verfahren gemäß EP 369 823 ist, daß die stickstoffhaltige Katalysatorkomponente verbraucht wird und nicht rückführbar ist und daher hohe Kosten verursacht.

Ein Nachteil der Verfahren gemäß EP 127 888 und EP 167 153 ist, daß der als Katalysator verwendete Kupfer-Alkalimetall-Halogen-Komplex extra vorher hergestellt werden muß.

Nachteilig an den Verfahren gemäß EP 127 888, EP 167 153 und EP 294 584 ist, daß die als am besten geeignet beschriebene Katalysatorkomponente LiCl sehr teuer ist und selbst bei sehr geringen Verlusten einen erheblichen Kostenfaktor darstellt.

Nachteilig an den vier zuletzt genannten Verfahren ist weiter, daß bei den als besonders geeignet beschriebenen Varianten die Reaktionstemperatur oberhalb bzw. am Flammpunkt des Lösungsmittels liegt und somit in technischen Anlagen beim Arbeiten mit Sauerstoff eine erhöhte Explosionsgefahr resultieren würde.

Es war daher die Aufgabe der Erfindung, ein Verfahren zur katalytischen Oxidation von TMP zu TMC zu entwickeln, bei dem der Katalysator möglichst kostengünstig ist und bei dem gleichzeitig gute Ausbeuten an TMC erhalten werden, der Katalysator leicht herstellbar und leicht aus dem Reaktionsgemisch rückgewinnbar ist, der Flammpunkt des Lösungsmittels oberhalb der Reaktionstemperatur liegt und außerdem der Siedepunkt des Lösungsmittels weit genug vom Siedepunkt des TMC entfernt ist, so daß eine einfache destillative Abtrennung und Reinigung des TMC sowie eine einfache Rückgewinnung des Lösungsmittels gewährleistet ist.

Es wurde nun überraschenderweise gefunden, daß sich die Aufgabe dieser Erfindung weitgehend lösen läßt, wenn man als Katalysatoren Kupfer(II)-halogenide verwendet, denen zur Aktivitätssteigerung Erdalkalihalogenide zugesetzt werden, und als Lösungsmittelsysteme ein Reaktionsmedium bestehend aus Wasser und einem gesättigten aliphatischen Alkohol mit 5 bis 10 C-Atomen bzw. aus Wasser und einem Gemisch aus einem gesättigten aliphatischen Alkohol mit 1 bis 4 C-Atomen und einem aromatischen Kohlenwasserstoff einsetzt.

Dieses Ergebnis war insofern nicht zu erwarten, da die Löslichkeit von Erdalkalimetallhalogeniden in organischen Lösungsmitteln deutlich geringer ist als die Löslichkeit von Alkalimetallhalogeniden. Daher waren niedrigere Katalysatorkonzentrationen in der organischen Phase und somit eine geringere Eignung für die genannte Reaktion zu erwarten.

Auch wurde in den neueren Patentschriften nie in Betracht gezogen, daß auch ein Katalysatorsystem bestehend aus einem Kupfer(II)-halogenid und einem Erdalkalimetallhalogenid für die gewünschte Umsetzung gut geeignet sein könnte, d.h. daß ein solches System bei der Vielzahl an durch-geführten Optimierungsversuchen somit als nicht geeignet angesehen werden mußte.

Überraschenderweise zeigte sich jedoch, daß die Oxidation von TMP zu TMC unter den genannten Bedingungen sehr vorteilhaft verläuft, und dies unter Erfüllung von technischen und ökonomischen Gesichtspunkten.

Durch Verwendung von Erdalkalimetallhalogeniden, insbesondere von $MgCl_2$, als aktivierende Katalysatorkomponente, lassen sich Ausbeuten von > 90 % erzielen, ähnlich wie bei Verwendung von LiCl, mit dem Vorteil, eine wesentlich kostengünstigere Katalysatorkomponente einsetzen zu können.

Gegenstand der Erfindung ist daher ein Verfahren zur Herstellung von 2,3,5-Trimethyl-p-benzochinon durch Oxidation von einem Trimethylphenol, insbesondere 2,3,6-Trimethylphenol, mit Sauerstoff oder einem sauerstoffhaltigen Gas in Gegenwart eines Kupfer(II)-halogenid enthaltenden Katalysators in einem zweiphasigen Reaktionsmedium bei erhöhter Temperatur, das dadurch gekennzeichnet ist, daß man die Umsetzung in einem Reaktionsmedium bestehend aus Wasser und einem gesättigten aliphatischen Alkohol mit 5 bis 10 C-Atomen oder aus Wasser und einem Gemisch aus einem Alkohol mit 1 bis 4 C-Atomen und einem aromatischen Kohlenwasserstoff und in Gegenwart von einem aus einem Kupfer(II)-halogenid und einem Erdalkalimetallhalogenid bestehenden Katalysatorsystem bei Temperaturen zwischen 40 und 100°C durchführt.

Als Lösungsmittel sind unter Berücksichtigung von technischen Aspekten, wie z.B. Abtrennbarkeit von Lösungsmittel und TMC (Siedepunkt 231°C) oder Abstand zwischen Reaktionstemperatur und Flammpunkt des Lösungsmittels, besonders Alkohole mit 7 bis 8 C-Atomen geeignet. In Tabelle 1 sind einige der erfindungsgemäß anwendbaren Alkohole mit den in ihnen bevorzugten Reaktionstemperaturen und den Flammpunkten zusammengefaßt.

Tabelle 1

| Alkohol | Flammpunkt | Siedepunkt | Bevorzugte Reaktionstemperatur |
|---|---|---|---|
| Methanol | 11 °C | 65 °C | 40-60 °C |
| Ethanol | 8 °C | 78 °C | 40-60 °C |
| 1-Propanol | 15 °C | 97 °C | 40-60 °C |
| Isopropanol | 22 °C | 82 °C | 40-60 °C |
| 1-Butanol | 35 °C | 118 °C | 40-60 °C |
| 2-Butanol | 26 °C | 98 °C | 40-60 °C |
| Isobutanol | 37 °C | 108 °C | 40-60 °C |
| tert.-Butanol | 4 °C | 83 °C | 40-60 °C |
| 1-Pentanol | 48 °C | 137 °C | 60 °C |
| 1-Hexanol | 60 °C | 157 °C | 60 °C |
| Cyclohexanol | 67 °C | 160 °C | 70 °C |
| 1-Heptanol | 73 °C | 176 °C | 70 °C |
| 1-Octanol | 81 °C | 196 °C | 70 °C |
| 2-Ethyl-1-hexanol | 76 °C | 185 °C | 70 °C |
| 1-Nonanol | 75 °C | 215 °C | 70 °C |
| 1-Decanol | 82 °C | 231 °C | 70 °C |

Da für ein industrielles Verfahren neben den technischen Gesichtspunkten, wie Abtrennbarkeit und Reinigung des Produkts oder eine gefahrlose Realisierung, auch ökonomische Aspekte berücksichtigt werden müssen, sollen die Einsatzstoffe möglichst kostengünstig sein. Daher ist 2-Ethyl-1-hexanol als ein billiger, großtechnisch verfügbarer Alkohol besonders geeignet.

Als gesättigte aliphatische Alkohole mit 5 bis 10 C-Atomen seien beispielsweise 1-Pentanol, 1-Hexanol, 1-Heptanol, 1-Octanol, 1-Nonanol, 1-Decanol, 2-Ethyl-1-hexanol, Cyclohexanol genannt, insbesondere 2-Ethyl-1-hexanol.

Als niedere gesättigte aliphatische Alkohole mit 1 bis 4 C-Atomen seien Methanol, Ethanol, 1-Propanol, Isopropanol, 1-Butanol, 2-Butanol, Isobutanol und tert.-Butanol genannt.

Als aromatische Kohlenwasserstoffe kommen vorzugsweise solche mit 6 bis 8 C-Atomen in Betracht, insbesondere Benzol, Toluol, Xylol oder Chlorbenzol.

Als geeignete Kupfer(II)-halogenide seien im wesentlichen $CuCl_2$ und $CuBr_2$, insbesondere $CuCl_2$, genannt.

Von den Erdalkalimetallhalogeniden sind im wesentlichen $MgCl_2$, $MgBr_2$, $CaCl_2$ und $CaBr_2$, insbesondere $MgCl_2$ und $CaCl_2$ geeignet.

Die beiden Komponenten des Katalysatorsystems, das Kupfer(II)-halogenid und das Erdalkalimetallhalogenid, werden dem Reaktionsgemisch in Form ihrer wäßrigen Lösung zugefügt oder aber beide in der wäßrigen Phase des Reaktionsgemisches gelöst. Die wäßrige, den Katalysator enthaltende Lösung ist also auf sehr einfache Weise herstellbar. Das aufwendige Vorfertigen eines Komplexkatalysators, wie es gemäß EP 127 888 und EP 167 153 beschrieben wird, ist nicht notwendig.

Die Konzentration des Kupfer(II)-halogenids in Wasser kann 5 bis 70 Gew.%, vorzugsweise 10 bis 50 Gew.%, betragen. Die Erdalkalimetallhalogenide werden bevorzugt in Konzentrationen von 5 bis 80 Gew.% eingesetzt.

Die Menge des Kupfer(II)-halogenids in Mol beträgt das 0,1- bis 10-fache, vorzugsweise das 0,4- bis 2-fache der eingesetzten molaren TMP-Menge. Die Erdalkalihalogenide werden in der 0,1- bis 10-fachen, vorzugsweise 0,5- bis 5-fachen molaren Menge bezogen auf TMP eingesetzt. Zusätzlich zu den Erdalkalimetallhalogeniden können auch noch andere aus dem Stand der Technik bekannte Aktivatoren, vor allem Kupfer(I)-Salze, eingesetzt werden.

Zur Durchführung der Reaktion wird das TMP in der organischen Komponente des Lösungsmittelsystems gelöst. Die Konzentration von TMP in der organischen Komponente liegt im allgemeinen zwischen 5 und 80, vorzugsweise zwischen 10 und 50 Gew.%. Das Mengenverhältnis von wäßriger Phase zu Alkoholphase kann im Bereich von 10:1 bis 1:10 schwanken, bevorzugt wird der Bereich von 3:1 bis 1:3.

Als Oxidationsmittel kann reiner Sauerstoff oder ein sauerstoffhaltiges Gas, wie Luft, eingesetzt werden.

Für die Zugabe der alkoholischen TMP-Lösung zur wäßrigen Katalysatorlösung gibt es 2 Möglichkeiten. Entweder wird die Gesamtmenge sofort zu Beginn der Reaktion zugefügt oder aber während der Reaktion langsam zugetropft.

Durch eine Reaktionsführung nach der zweiten Variante (semi-batch-Prozeß) läßt sich die Ausbeute von TMC optimieren, da die Bildung von Nebenprodukten, wie Hexamethylbiphenol, weitgehend unterdrückt wird.

Die Reaktionstemperatur ist prinzipiell nicht auf einen engen Bereich limitiert. Sie liegt bevorzugt zwischen 40 und 100°C.

Die folgenden Beispiele sollen die Erfindung verdeutlichen, ohne sie einzuengen.

Beispiele 1 bis 4

In einem 500-ml-Glaskolben wurde jeweils eine Lösung der aus Tabelle 2 ersichtlichen Mengen an Kupfer(II)halogenid und Erdalkalimetallhalogenid in 100 g Wasser vorgelegt, die Lösung mit einem Wasserbad auf die in Tabelle 2 angegebene Temperatur erwärmt und der Gasraum der Apparatur 2mal evakuiert und mit Sauerstoff belüftet. Anschließend wurde eine Lösung von jeweils 34 g TMP (0.25 Mol) in 100 g des in Tabelle 2 angegebenen Alkohols entweder sofort vollständig hinzugefügt oder im Verlauf von 2 bis 3 Stunden zur Katalysatorlösung hinzugetropft. Dabei wurde unter Einhaltung der angegebenen Temperatur das Reaktionsgemisch mit einem Flügelrührer mit 800-1100 U/min gerührt und aus einer Gasbürette Sauerstoff in den Gasraum des Glaskolbens geleitet, so daß der Verlauf der Reaktion am Sauerstoffverbrauch verfolgt werden konnte. Nach Beendigung der Sauerstoffaufnahme wurde die organische Phase von der wäßrigen Phase abgetrennt. Die organische Phase wurde 2mal bei 60 bis 70°C mit Wasser gewaschen und die Ausbeute durch Gaschromatographie mit internem Standard ermittelt. Der TMP-Umsatz lag in allen Fällen bei 100 %.

Tabelle 2

| Beispiel Nr. | Alkohol | Reaktions-temperatur [°C] | Katalysator (Molmenge) | TMP/Alkohol-Zugabezeit [Min] | $O_2$-Aufnahme-zeit [Min] | TMC-Ausbeute [% der Theorie] |
|---|---|---|---|---|---|---|
| 1 | 2-Ethyl-1-hexanol | 70 | $CuCl_2$ (0.25) $MgCl_2$ (0.5) | 120 | 180 | 98.5 |
| 2 | 2-Ethyl-1-hexanol | 70 | $CuCl_2$ (0.25) $MgCl_2$ (0.5) | sofort | 60 | 87.5 |
| 3 | 1-Hexanol | 60 | $CuCl_2$ (0.25) $MgCl_2$ (0.5) | 180 | 220 | 97.0 |
| 4 | 1-Hexanol | 60 | $CuCl_2$ (0.25) $MgCl_2$ (0.5) | sofort | 60 | 86.5 |

**Patentansprüche**

1. Verfahren zur Herstellung von 2,3,5-Trimethyl-p-benzochinon durch Oxidation von einem Irimethylphenol mit Sauerstoff oder einem sauerstoffhaltigen Gas in Gegenwart eines Kupfer(II)-halogenid enthalten-

6

den Katalysators in einem zweiphasigen Reaktionsmedium bei erhöhter Temperatur, dadurch gekennzeichnet, daß man die Umsetzung in einem Reaktionsmedium bestehend aus Wasser und einem gesättigten aliphatischen Alkohol mit 5 bis 10 C-Atomen oder aus Wasser und einem Gemisch aus einem gesättigten aliphatischen Alkohol mit 1 bis 4 C-Atomen und einem aromatischen Kohlenwasserstoff und in Gegenwart von einem aus einem Kupfer(II)-halogenid und einem Erdalkalimetallhalogenid bestehenden Katalysatorsystem bei Temperaturen zwischen 40 und 100°C durchführt.

2. Verfahren zur Herstellung von 2,3,5-Trimethyl-p-benzochinon gemäß Anspruch 1, dadurch gekennzeichnet, daß man die Umsetzung in einem Reaktionsmedium bestehend aus Wasser und 2-Ethyl-1-hexanol durchführt.

3. Verfahren zur Herstellung von 2,3,5-Trimethyl-p-benzochinon gemäß Anspruch 1, dadurch gekennzeichnet, daß man die Umsetzung in einem Reaktionsmedium bestehend aus Wasser und einem Gemisch aus einem $C_1$- bis $C_4$-Alkohol mit Benzol oder Toluol durchführt.

4. Verfahren zur Herstellung von 2,3,5-Trimethyl-p-benzochinon gemäß Anspruch 1, dadurch gekennzeichnet, daß man als Erdalkalimetallhalogenid Magnesiumchlorid oder Calciumchlorid verwendet.

5. Verfahren zur Herstellung von 2,3,5-Trimethyl-p-benzochinon gemäß Anspruch 1, dadurch gekennzeichnet, daß man die alkoholische Lösung von Trimethylphenol bzw. die Lösung von Trimethylphenol in einem Gemisch aus einem Alkohol und einem aromatischen Kohlenwasserstoff während der Reaktion langsam in die wäßrige Katalysatorlösung eintropfen läßt.

**Claims**

1. A process for preparing 2,3,5-trimethyl-p-benzoquinone by oxidation of a trimethylphenol with oxygen or an oxygen-containing gas in the presence of a catalyst containing copper (II) halide in a two-phase reaction medium at elevated temperature, wherein the reaction is carried out in a reaction medium composed of water and a saturated aliphatic alcohol with 5 to 10 C atoms or of water and a mixture of a saturated aliphatic alcohol with 1 to 4 C atoms and an aromatic hydrocarbon and in the presence of a catalyst system composed of a copper(II) halide and an alkaline earth metal halide at from 40 to 100°C.

2. A process for preparing 2,3,5-trimethyl-p-benzoquinone as claimed in claim 1, wherein the reaction is carried out in a reaction medium composed of water and 2-ethyl-1-hexanol.

3. A process for preparing 2,3,5-trimethyl-p-benzoquinone as claimed in claim 1, wherein the reaction is carried out in a reaction medium composed of water and a mixture of a $C_1$-$C_4$-alcohol with benzene or toluene.

4. A process for preparing 2,3,5-trimethyl-p-benzoquinone as claimed in claim 1, wherein magnesium chloride or calcium chloride is used as alkaline earth metal halide.

5. A process for preparing 2,3,5-trimethyl-p-benzoquinone as claimed in claim 1, wherein an alcoholic solution of trimethylphenol or a solution of trimethylphenol in a mixture of an alcohol and an aromatic hydrocarbon is slowly added dropwise to the aqueous catalyst solution during the reaction.

**Revendications**

1. Procédé de préparation de 2,3,5-triméthyl-p-benzoquinone par oxydation d'un triméthylphénol avec de l'oxygène ou un gaz contenant de l'oxygène, en présence d'un catalyseur contenant un halogénure de cuivre (II), dans un milieu réactionnel à deux phases et à température élevée, caractérisé en ce qu'on conduit la réaction dans un milieu réactionnel composé d'eau et d'un alcool aliphatique saturé à 5-10 atomes de carbone ou composé d'eau et d'un mélange d'un alcool aliphatique saturé à 1-4 atomes de carbone et d'un hydrocarbure aromatique, et en présence d'un système catalytique composé d'un halogénure de cuivre (II) et d'un halogénure de métal alcalino-terreux, à une température comprise entre 40 et 100°C.

2. Procédé de préparation de 2,3,5-triméthyl-p-benzoquinone selon la revendication 1, caractérisé en ce qu'on conduit la réaction dans un milieu réactionnel composé d'eau et de 2-éthyl-1-hexanol.

3. Procédé de préparation de 2,3,5-triméthyl-p-benzoquinone selon la revendication 1, caractérisé en ce qu'on conduit la réaction dans un milieu réactionnel composé d'eau et d'un mélange d'un alcool en $C_1$-$C_4$ et de benzène ou de toluène.

4. Procédé de préparation de 2,3,5-triméthyl-p-benzoquinone selon la revendication 1, caractérisé en ce qu'on utilise, comme halogénure de métal alcalino-terreux, du chlorure de magnésium ou du chlorure de calcium.

5. Procédé de préparation de 2,3,5-triméthyl-p-benzoquinone selon la revendication 1, caractérisé en ce qu'au cours de la réaction, on introduit goutte à goutte et lentement, dans la solution aqueuse de catalyseur, la solution alcoolique de triméthylphénol ou la solution de triméthylphénol dans un mélange d'un alcool et d'un hydrocarbure aromatique.